# EUROPÄISCHE PATENTANMELDUNG

(11) **EP 0 670 163 A1**
(43) Veröffentlichungstag der Anmeldung: **06.09.1995**
(21) Anmeldenummer: 94112421.6
(22) Anmeldetag: 09.08.1994
(51) Int. Cl.: A61K 31/52

(54) **Verwendung von Pentoxifyllin zur Herstellung pharmazeutischer Zubereitungen für die Behandlung granulomatöser und fibrosierender Lungenerkrankungen**

(30) Priorität: 21.09.1993 DE 4332041
(71) Anmelder: Dr. Rentschler Arzneimittel GmbH & Co., D-88471 Laupheim (DE)
(72) Erfinder: Zabel, Peter, Dr., D-23795 Bad Segeberg (DE); Schade, Ulrich, Dr., D-23821 Wardersee (DE)
(74) Vertreter: Schwabe - Sandmair - Marx

(57) **Zusammenfassung**

Die Erfindung betrifft die Verwendung von Pentoxifyllin, gegebenenfalls in Kombination mit weiteren therapeutischen Wirkstoffen zur Herstellung pharmazeutischer Zubereitungen für die Behandlung von granulomatösen und fibrosierenden Lungenerkrankungen.

## Beschreibung

Die Erfindung betrifft die Verwendung von Pentoxifyllin zur Behandlung von granulomatösen und fibrosierenden Lungenerkrankungen.

Bei der Lungengranulomatose handelt es sich um eine krankhafte Bindegewebsvermehrung im Lungengerüst, die durch eine Einwirkung verschiedener Noxen auf die Lunge verursacht wird. Als Noxen für die Auslösung einer granulomatösen Lungenerkrankung sind Mykobakterien, Immunkomplexe, Autoantikörper sowie eine Reihe weiterer Substanzen bekannt. Demgegenüber ist bei der Sarkoidose der Stimulus unbekannt. Bei der Sarkoidose handelt es sich um eine systemische granulomatöse Erkrankung ungeklärter Ätiologie, die obligat zu granulomatösen, interstitiellen Veränderungen in den Lungen und hilären Lymphknoten führt. Fakultativ können alle Organe, bevor zugt ZNS, Leber, Herz, Augen, Haut und Knochen, in den systemischen Krankheitsprozeß einbezogen sein. Die pulmonalen Veränderungen können bei chronischer Prozeßaktivität in eine irreversible Lungenfibrose mit bleibenden Organschaden übergehen.

Zur Therapie vorgenannter Lungenerkrankungen ist nach dem Stand der Technik eine symptomatische, systemische Corticosteroidtherapie das Mittel der Wahl. Da es sich um eine Corticosteroidlangzeittherapie mit Dosen oberhalb der "Cushingschwelle" handelt, kann es zu den üblichen corticosteroid-bedingten Nebenwirkungen kommen. Im Falle der fibrosierenden Lungenerkrankungen wird nach dem Stand der Technik ebenfalls eine Corticosteroidtherapie entweder allein oder in Verbindung mit weiteren Immunsupressiva und Zytostatika je nach Ätiologie empfohlen. Insgesamt ist dazu aber festzustellen, daß ein hoher Prozentsatz der Lungenfibrosen auf keine der genannten Therapieformen anspricht oder es bedarf einer Langzeittherapie mit den genannten Substanzen mit entsprechenden gravierenden Nebenwirkungen. Die im Rahmen einer systemischen Sklerodermie auftretenden Lungenfibrose spricht regelmäßig auf keine der bisherigen Therapien an, so daß bei der Sklerodermie die progrediente Fibrosierung der Lunge zum Tode führt.

Die Anmelderin hat nun gefunden, daß Pentoxifyllin zur Behandlung vorgenannter Erkrankungen geeignet ist, ohne dabei die mit der bislang üblichen Therapie verbundenen Nebenwirkungen zu zeigen.

Pentoxifyllin ist als Vasodilatator bekannt. Eine Verwendung zur Behandlung der hier genannten Krankheiten ist bislang nicht bekannt.

Im Rahmen einer offenen Therapiestudie wurde von der Anmelderin gefunden, daß bei Patienten mit einer bronchoskopisch nachgewiesenen intrathorakalen Sarkoidose in 80 % der Fälle die Patienten auf eine entsprechende Pentoxifyllinbehandlung, 4 bis 5 x 400 mg oral/Tag für 3 bis 6 Monate ansprachen. Es zeigte sich eine Rückläufigkeit der radiologischen Veränderungen im Bereich der Lungen und hilären Lymphknoten oder eine Besserung bis zur Normalisierung der Lungenfunktionsparameter. In etwa 30 % der Fälle kam es nach Beendigung der Pentoxifyllintherapie nach 6 Monaten zu einer erneuten Krankheitsaktivität, die bei Wiederbeginn der Pentoxifyllintherapie erneut sistierte. Lediglich in 16 % der Fälle wurde festgestellt, daß kein Anspruch auf Pentoxifyllin erfolgte, und erst durch eine Kombinationstherapie, bestehend aus Pentoxifyllin und Corticosteroiden, eine radiologische und lungenfunktionelle Besserung mit anhaltender Remission erzielt werden konnte.

Die eingesetzte Dosis Pentoxifyllin hängt natürlich von der Schwere der Erkrankung, vom Zustand und vom Gewicht des zu behandelnden Patienten ab. Sie beträgt jedoch im allgemeinen 1,00 - 3,00 g/Tag und vorzugsweise 1,20 - 2,50 g/Tag.

Pentoxifyllin kann, wie bereits gesagt, auch in Kombination mit Corticosteroiden eingesetzt werden. Bevorzugte Corticosteroide sind Prednison, Prednisolon, Prednisolon-21-Acetat, Prednyliden und Methylprednison. Die Konzentration des eingesetzten Corticosteroids beträgt in der Initialphase (ca. 2 Wochen) 0,3 - 1,2 mg, vorzugsweise, 0,5 - 1,0 mg/kg Körpergewicht. Nach der Initialphase erfolgt eine langsame Dosisreduktion auf Erhaltungsdosen von 2,5 - 7,5 mg/Tag, vorzugsweise 10 - 20 mg/Tag.

Die Verabreichung der Zubereitung erfolgt vorzugsweise auf oralem Weg, wobei auch andere Verabreichungsformen möglich sind.

### Beispiele:

Beispiel 1: Ein männlicher Patient (51 Jahre, 88 kg W.L.) mit seit 7 Jahren bekannter progressiver systemischer Sklerodermie (Raynaud Phänomen an den Akren mit Nekrosen an Fingern und Zehen, Schluckstörungen bei Ösophagusbeteiligung, Lungenfibrose mit hochgradiger respiratorischer Partialinsuffizienz) war ohne Effekt mit verschiedenen immunsuppressiven Therapieschemata behandelt worden (hochdosierte Corticosteroide mit Cyclophosphamid, Plasmaseperationen mit Cyclophosphamid-Stoßtherapie, Corticosteroiden mit Cyclosporin-A). Nach 3-monatiger Kombination von Pentoxifyllin (5x 400 mg oral/Tag) mit Corticosteroiden in absteigender Dosis bis zu einer Erhaltungstherapie von 10 mg Methylprednisolon oral/Tag zeigte sich ein deutlicher radiologischer Befundregreß der fibrosierenden Lungenveränderungen, eine Normalisierung der Blutgase in Ruhe, ein Verschwinden der Schluckstörungen und eine Abheilung der Nekrosen im Bereich der Akren. Bisher hält die Remission über 1 Jahr an unter Fortsetzung der genannten Kombinationstherapie.

Beispiel 2: Ein männlicher Patient (45 Jahre, 61 kg R.S.) mit seit 6 Jahren bekannter progressiver systemischer Sklerodermie (Schluckstörungen bei Ösophagusbeteiligung, Lungenfibrose mit respiratorischer Globalinsuffizienz (Sauerstofflangzeittherapie) sprach ebenfalls auf die verschiedensten immunsuppressiven Kombinationsstrategien nicht an. Unter einer Kombination von Pentoxifyllin (4 x 400 mg oral/Tag) mit Corticosteroiden in absteigender Dosierung bis zu einer Dosis von 20 mg Methylprednisolon oral/Tag zeigte sich nach 6 Monaten Therapie eine signifikante Besserung der Lungenfunktionsparameter (respiratorischer Partialinsuffizienz).

Beispiele 3-7: 5 Patienten (weiblich; 28 Jahre, C.W.; weiblich, 45 Jahre, D.H., männlich, 48 Jahre, K.K.; männlich, 24 Jahre, G.H.; männlich, 42 Jahre, J.S.) zeigten ein Therapieansprechen, das durch Rückläufigkeit der radiologischen Veränderungen im Bereich von Lungen und hilären Lymphknoten und/oder Besserung bis Normalisierung der Lungenfunktionsparameter dokumentierbar war. Die Patienten erhielten nur Pentoxifyllin in einer Dosierung von 4 x 400 mg oral/Tag.

Beispiel 8: Ein Patient (männlich, 31 Jahre, H.G.) zeigte auf Pentoxifyllin kein Ansprechen der Krankheitsaktivität, so daß auf eine orale Corticosteroidtherapie gewechselt wurde. Als auch diese nach 6 Wochen keinen Effekt zeigte, wurde er mit einer Kombination aus Pentoxifyllin mit Corticosteroiden behandelt (4 x 400 mg Pentoxifyllin oral/Tag und 60 mg Methylprednisolon oral/Tag). Nach 2 Wochen zeigte sich eine radiologische und lungenfunktionell dokumentierbare Besserung, so daß schrittweise die Corticosteroiddosis bis auf eine Erhaltungsdosis 10 mg/Tag reduziert werden konnte. Die Remission hielt nach 9 Monaten noch an.

Beispiele 9 und 10: Bei 2 Patienten (weiblich, 28 Jahre, C.W.; weiblich, 45 Jahre, D.H.) kam es nach Beendigung der Pentoxifyllintherapie nach 6 Monaten zu einer erneuten Krankheitsaktivität, die bei Wiederbeginn der Pentoxifyllin-Therapie mit 4 x 400 mg/Tag erneut sistierte. Beide Patienten erhielten zusätzlich 10 mg Prednison/Tag, das oral verabreicht wurde.

## Patentansprüche

1. Verwendung von Pentoxifyllin zur Herstellung pharmazeutischer Zubereitungen für die Behandlung von granulomatösen und fibrosierenden Lungenerkrankungen.

2. Verwendung nach Anspruch 1, in Kombination mit einem oder mehreren weiteren therapeutischen Wirkstoffen.

3. Verwendung nach Anspruch 2, dadurch gekennzeichnet, daß der weitere therapeutische Wirkstoff ein Corticosteroid aus der Gruppe der nicht fluorierten Glukocorticoide ist.
